(19) Europäisches Patentamt
European Patent Office
Office européen des brevets

(11) **EP 2 940 508 A1**

(12) **EUROPEAN PATENT APPLICATION**

(43) Date of publication:
**04.11.2015 Bulletin 2015/45**

(51) Int Cl.:
***G02B 23/24*** *(2006.01)*   ***G01N 21/954*** *(2006.01)*
***A61B 1/00*** *(2006.01)*

(21) Application number: **15165769.9**

(22) Date of filing: **29.04.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **30.04.2014 CN 201410180922**

(71) Applicant: **General Electric Company Schenectady, NY 12345 (US)**

(72) Inventors:
• **Gu, Jiajun**
**201203 Shanghai (CN)**
• **Yang, Yong**
**201203 Shanghai (CN)**
• **Han, Jie**
**201203 Shanghai (CN)**

• **Zhai, Zirong**
**201203 Shanghai (CN)**
• **Harding, Kevin George**
**Niskayuna, NY New York 12309 (US)**
• **Song, Guiju**
**Niskayuna, NY New York 12309 (US)**
• **Jia, Ming**
**201203 Shanghai (CN)**
• **Xie, Guangping**
**201203 Shanghai (CN)**
• **Lim, Ser Nam**
**Niskayuna, NY New York 12309 (US)**

(74) Representative: **Cleary, Fidelma**
**GPO Europe**
**GE International Inc.**
**The Ark**
**201 Talgarth Road**
**Hammersmith**
**London W6 8BJ (GB)**

(54) **BORESCOPE AND NAVIGATION METHOD THEREOF**

(57) The borescope includes an insertion tube, a first image processor (304), a model store unit (306), a pose calculator (307), a second image processor (314), a navigation image calculator (310) and a display (220). The insertion tube includes a detection head (242) and at least one sensor (2465) for receiving signals in the insertion tube and generating sensed signals (316). The first image processor (304) is for calculating a first image (312) based on first image signals (311) captured by the detection head (242). The second image processor (314) is for adjusting the initial pose (317) calculated by the pose calculator (307) to a navigation pose (324) until a difference between the first image (312) and a second image (322) calculated based on the navigation pose (324) and a predetermined model (318) falls in an allowable range. The navigation image calculator (310) is for calculating a navigation image (325) based on the navigation pose (324) and the predetermined model (318). The display (220) is for showing the navigation image (325).

FIG. 4

**Description**

BACKGROUND

Embodiments of the invention relate generally to borescopes and more particularly to a borescope having an accurate position tracking function for a detection head of the borescope in a mechanical device to be detected, and a navigating method thereof.

Borescopes are commonly used in the visual inspection of a mechanical device such as aircraft engines, industrial gas turbines, steam turbines, diesel engines, and automotive and truck engines. Gas and steam turbines require particular inside attention because of safety and maintenance requirements.

A flexible borescope is more commonly used in inspecting a complex interior surface of the mechanical device. In some cases, a detection head is assembled at a distal end of a flexible insertion tube of the borescope. More specifically, the detection head may include a miniature video camera and a light for making it possible to capture video or still images deep within dark spaces inside of the mechanical device. As a tool for remote visual inspection, the ability to capture video or still images for subsequent inspection is a huge benefit. A display in a hand-held operation apparatus shows the camera view, and a joystick control or a similar control is operated to control or steer the motion of the detection head for a full inspection of the interior elements of the mechanical device.

However, a full inspection usually takes several days to cover every region of interest (ROI) in the detected mechanical device through applying a borescope. One of the challenges that causes this long inspection time is the difficulty in navigating the detection head (borescope tip) to some ROI and navigating the miniature video camera of the detection head in a desired direction. The navigating view is the most important information available to the operator to judge the position of the detection head. Under some circumstances, an accurate pose (position and orientation) of the detection head in the detected mechanical device is desired in order to predict potential failure at the detected location and for the operator's reference for further operation.

For these and other reasons, there is a need for providing a new borescope and a navigating method thereof which can capture accurate position information of a detection head of the borescope, to better navigate the detection head.

BRIEF DESCRIPTION

In accordance with an embodiment of the invention, a borescope is provided. The borescope includes an insertion tube, a first image processor, a model store unit, a pose calculator, a second image processor, a navigation image calculator and a display. The insertion tube includes a detection head and at least one sensor for receiving signals in the insertion tube and generating sensed signals. The first image processor is for calculating a first image based on first image signals captured by the detection head. The model store unit is for storing a predetermined model of a mechanical device to be detected. The pose calculator is for calculating an initial pose of the detection head based on the sensed signals. The second image processor is for adjusting the initial pose to a navigation pose until a difference between the first image and a second image calculated based on the navigation pose and the predetermined model falls in an allowable range. The navigation image calculator is for calculating a navigation image based on the navigation pose and the predetermined model. The display is for showing the navigation image.

In accordance with another embodiment of the invention, A method for navigating a detection head of a borescope is provided. The method includes receiving first image signals from the detection head and sensed signals from at least one sensor. The method includes calculating an initial pose of the detection head based on the sensed signals. The method includes calculating a first image based on the first image signals and an initial second image based on the initial pose and a predetermined model. The method includes calculating an initial difference between the first image and the initial second image. The method includes adjusting the initial pose to a navigation pose gradually until a difference between the first image and a second image calculated based on the navigation pose and the predetermined model falls in an allowable range. The method includes calculating a navigation image based on the predetermined model and the navigation pose. The method includes showing the navigation image.

DRAWINGS

These and other features, aspects, and advantages of the present invention will become better understood when the following detailed description is read with reference to the accompanying drawings in which like characters represent like parts throughout the drawings, wherein:

FIG. 1 is a schematic view of a borescope used in an inspection operation for a mechanical device in accordance with one exemplary embodiment;

FIG. 2 is a cross-sectional view of an insertion tube of the borescope of FIG. 1 in accordance with one exemplary embodiment;

FIG. 3 is a schematic view of a sensing lead arranged in a shape sensing cable of the insertion tube of FIG. 2 in accordance with one exemplary embodiment;

FIG. 4 is a block diagram of the borescope of FIG. 1 in accordance with one exemplary embodiment;

FIG. 5 is a schematic view of an adjustment process of an initial second image in accordance with one exemplary embodiment;

FIG. 6 is a flowchart of a method for navigating a detection head of the borescope of FIG. 1 in accordance with one exemplary embodiment;

FIG. 7 is a flowchart of a step for adjusting an initial pose of the method of FIG. 6 in accordance with one exemplary embodiment; and

FIG. 8 is a flowchart of a step for adjusting the initial pose of the method of FIG. 6 in accordance with another exemplary embodiment.

DETAILED DESCRIPTION

Unless defined otherwise, technical and scientific terms used herein have the same meaning as is commonly understood by one of ordinary skill in the art to which this invention belongs. The terms "first", "second", and the like, as used herein do not denote any order, quantity, or importance, but rather are used to distinguish one element from another. Also, the terms "a" and "an" do not denote a limitation of quantity, but rather denote the presence of at least one of the referenced items, unless otherwise noted, are merely used for convenience of description, and are not limited to any one position or spatial orientation.

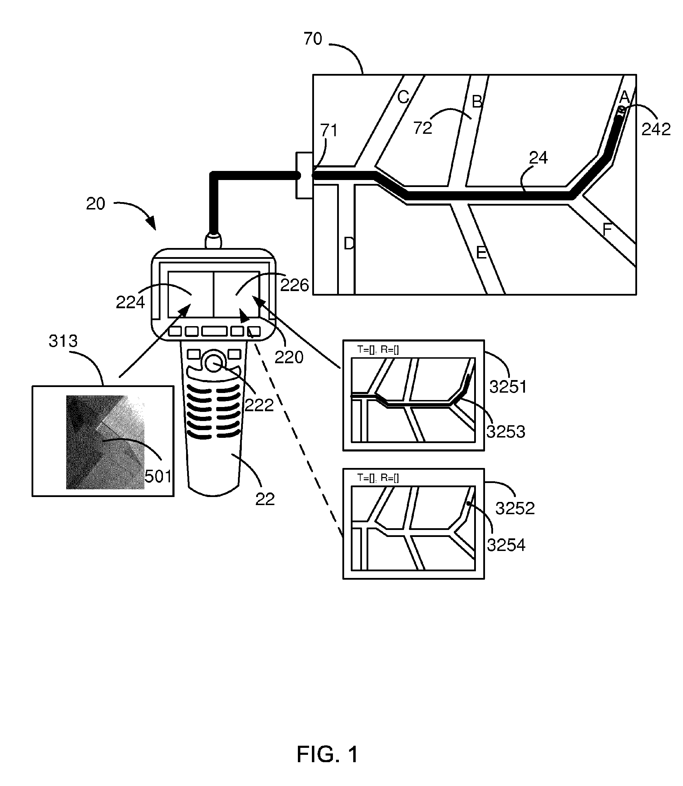

Referring to FIG. 1, a schematic view of a borescope 20 used in an inspection operation for a mechanical device 70 in accordance with one exemplary embodiment is shown. The borescope 20 includes a flexible insertion tube 24 and a hand-held operation apparatus 22. In some embodiments, a plurality of apertures (e.g., an aperture 71) is defined at some appropriate positions on the surface of the mechanical device 70. When detecting internal elements of the mechanical device 70, the inserting tube 24 is inserted into the detection space 72 of the mechanical device 70 through the aperture 71. As an example, the detection space 72 may have lots of channels, such as channels 'A', 'B', 'C', 'D', 'E', 'F' shown in FIG. 1.

In some non-limiting embodiments, the insertion tube 24 includes a detection head 242 at a distal end thereof. The detection head 242 may include a miniature video camera (not labeled) for capturing video or still images of the internal elements of the mechanical device 70. In some embodiments, the detection head 242 may include a light (not labeled) which makes it possible to capture video or still images deep within dark spaces of the mechanical device 70.

The hand-held operation apparatus 22 includes an operation part 222 (e.g., joystick control) for at least controlling or steering the motion of the detection head 242. The hand-held operation apparatus 22 further includes a display 220 which may include a first display part 224 for showing a corresponding video or still image 313 of an internal element 501 captured by the miniature video camera and a second display part 226 for showing a navigation image 3251 or 3252 of the detection head 242 in the mechanical device 70. In some embodiments, a construction 3253 of the insertion tube 24 and/or a navigation pose (e.g., position T and orientation R) of the detection head 242 are shown in the navigation image 3251. In some embodiments, only a point 3254 representing the detection head 242 and the information (T, R) of the detection head 242 are shown in the navigation image 3252. The navigation images 3251 and 3252 can be shown in two-dimension or in three-dimension views. The position T and the orientation R are three dimension vectors, for example, T=[Tx', Ty', Tz'], R=[Rx', Ry', Rz'] in the spatial coordinate system (x', y', z'). Although a hand-held operation apparatus 22 is shown for purposes of example, any type of apparatus enabling the functions described herein may be used.

Usually, an accurate navigation pose of the detection head 242 in the mechanical device 70 to be detected is desired in order to predict potential failure at the detected position and provide reference for the operator's subsequent operation.

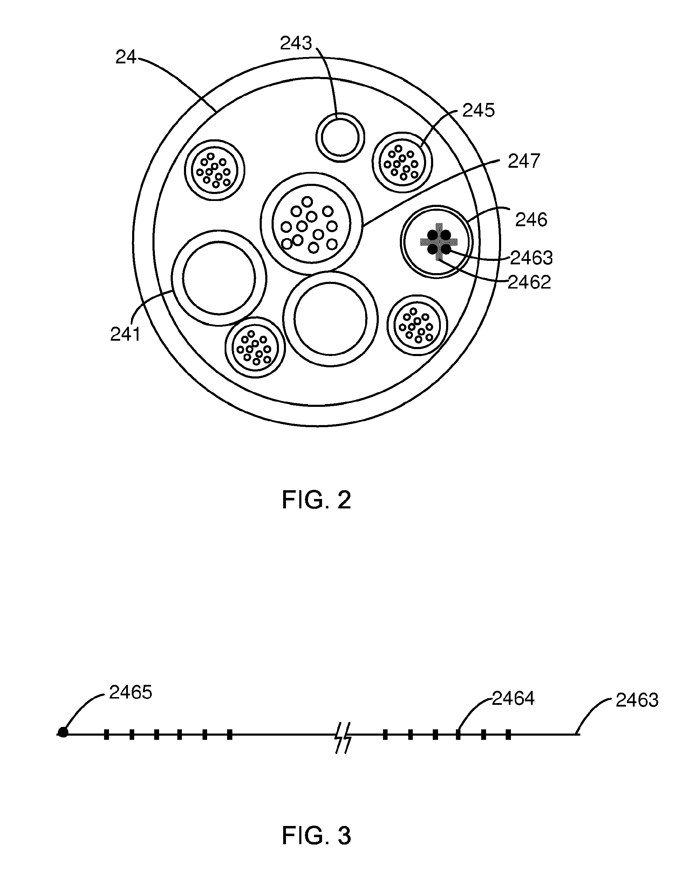

Referring to FIG. 2, a cross-sectional view of the insertion tube 24 of the borescope 20 in accordance with one exemplary embodiment is shown. For implementing a viewing function, the insertion tube 24 may include some cables therein, such as two lighting cables 241 for providing lighting source, a working channel 243 for transmitting camera signals, four articulation cables 245 for bending the detection head 242, and a power cable 247 for providing power.

The above cables are conventional technology and thus not described in detail. For implementing a navigation function, the insertion tube 24 further includes a shape sensing cable 246 arranged in the insertion tube 24. In this illustrated embodiment, the shape sensing cable 246 is arranged on an inner surface of the insertion tube 24. In other embodiments, the shape sensing cable 246 can be arranged in other positions of the insertion tube 24, for example arranged in the center of the insertion tube 24 which may obtain an optimal shape feedback of the insertion tube 24.

As an example, the shape sensing cable 246 includes a cross-shaped flexible support rod 2462 and four sensing leads 2463 respectively arranged four corners of the support rod 2462. In one embodiment, the support rod 2462 may be made of, but not limited to glass fiber or carbon fiber. The shape of the support rod 2462 can be changed, and the number of the sensing leads 2463 can be adjusted in other embodiments.

Referring to FIG. 3, a schematic view of a sensing lead 2463 arranged in a shape sensing cable 246 of the insertion tube of FIG. 2 in accordance with one exemplary embodiment is shown. The sensing lead 2463 includes a sensor 2465 and a plurality of sensing points 2464. In non-limiting embodiments, the sensing lead 2463 may comprise an optical fiber, and the at least one sensing point 2464 may include a Fiber Bragg Grating (FBG). The number of the sensing points 2464 is determined based on the precision requirement of the navigation function. In some embodiments, the sensor 2465 is attached to an end of the sensing lead 2463 for receiving the light signals reflected back at each sensing point 2464 and generating sensed signals for calculating the shape of the insertion tube 24 based on appropriate algorithms.

In some embodiments, each sensing point 2464 may include a strain gage or other piezo material based sensor. Each sensor at each sensing point 2464 is used to receive a strain change signal and generating sensed signals for calculating the shape of the insertion tube 24 based on appropriate algorithms.

In other embodiments, the sensor 2465 includes an accelerator and a gyroscope which are attached to the detection head 242. Therefore, the pose of the detection head 242 can be calculated based on sensed signals of the accelerator and the gyroscope by implementing inertial based navigation algorithms.

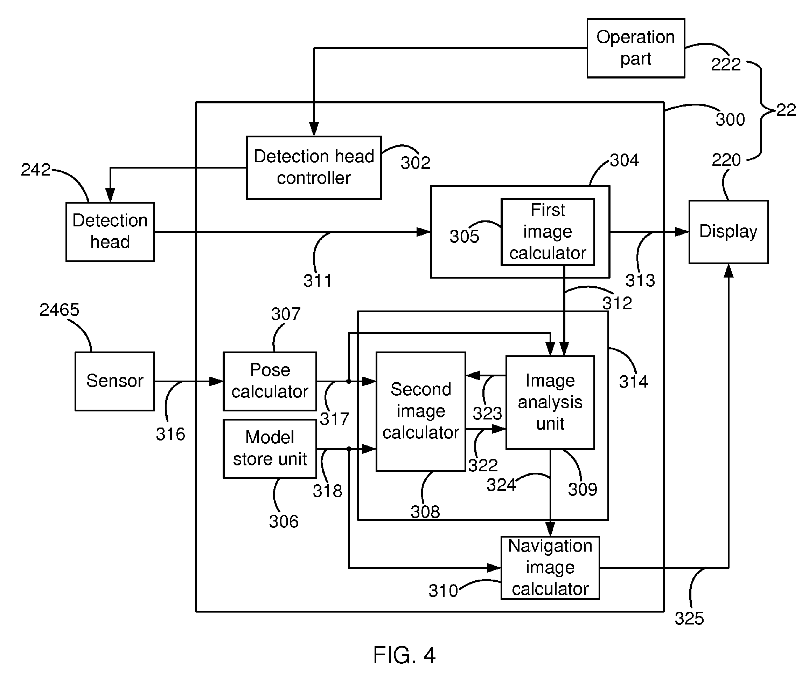

Referring to FIG. 4, a block diagram of the borescope 20 of FIG. 1 in accordance with one exemplary embodiment is shown. As mentioned above, the hand-held operation apparatus 22 is used to control the detection head 242 through the operation part 222 and show the captured video or still image 313 and a calculated navigation image 325 of the detection head 242 through the display 220. In some embodiments, the hand-held operation apparatus 22 may include a processor 300 embedded therein and in communication with the operation part 222, the display 220, the detection head 242, and the sensor 2465. In some embodiments, the processor 300 may be arranged in an external processing device (e.g., a computer) so as to minimize the size and weight of the hand-held operation apparatus 22.

For implementing the video or image viewing function, the processor 300 includes a detection head controller 302 and a first image processor 304. The detection head controller 302 is used to receive control signals from the operation part 222 and control the detection head 242 in the detection process, such as adjusting imaging angle, forward direction, and lighting grade, etc.

After capturing the image of the internal element 501 as shown in FIG. 1, the working channel 243 as shown in FIG. 2 is used to transmit corresponding first image signals 311 to the first image processor 304. The first image processor 304 is used to receive the first image signals 311 and then calculate the corresponding video or still image 313 for optionally showing them in the first display part 224 of the display 220.

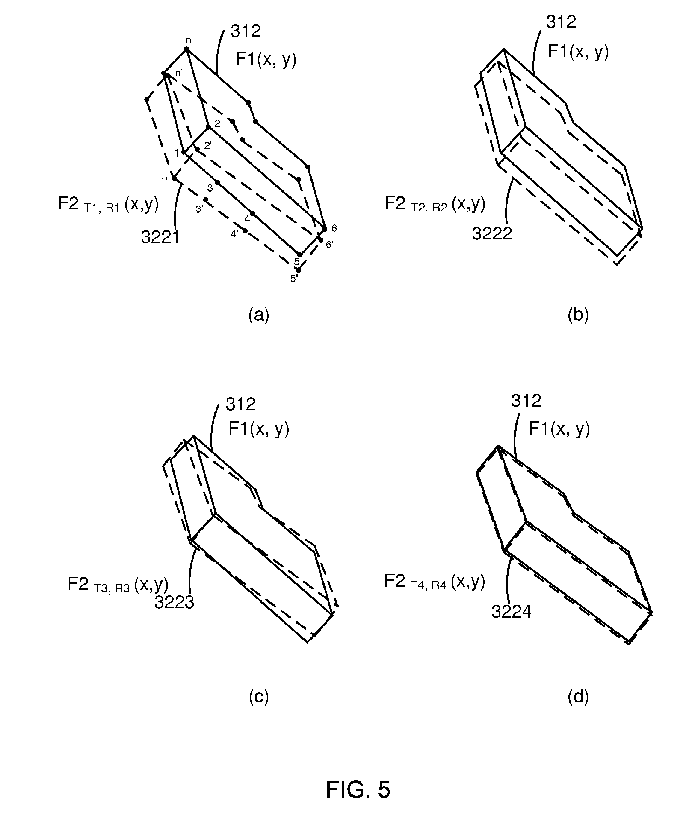

The first image processor 304 further includes a first image calculator 305 for calculating a first image $F1(x, y)$ 312 based on the first image signals 311. Herein, $F1(x, y)$ is a function in the planar coordinate system $(x, y)$. Referring to FIG. 5, as shown in the (a) part of FIG. 5, the first image $F1(x, y)$ 312 may include multiple feature points (e.g., 1, 2, 3,...n) of the captured internal element 501. In some embodiments, the feature points include a plurality of edge points which can be calculated by appropriate algorithms such as the gradient operator algorithm. In some embodiments, the feature points may include other points and lines or any other geometrical information that can be used to construct the image of the internal element 501.

Referring back to FIG. 4, for implementing the navigation function, the processor 300 further includes a model store unit 306, a pose calculator 307, a second image processor 314, and a navigation image calculator 310.

The model store unit 306 is used to store a predetermined model 318 which is determined according to the detected mechanical device 70. Namely, the configuration of the predetermined model 318 is the same as the configuration of the detected mechanical device 70. The model store unit 306 may store many models corresponding to different kinds of mechanical devices to be detected. In some embodiments, the predetermined model 318 may be a two-dimensional or a three-dimensional model.

The pose calculator 307 is used to receive the sensed signals 316 from the at least one sensor 2465 and calculate an initial pose 317 of the detection head 242 based on the sensed signals 316. The initial pose 317 includes an initial position T1 and an initial orientation R1 of the detection head 242 in the detected device 70. Usually, the initial pose (T1, R1) 317 of the detection head 242 is not accurate enough due to an error accumulation with the inserting length of the insertion tube 24 in the mechanical device 70. Therefore, the initial pose (T1, R1) 317 needs to be adjusted in order to navigate the detection head 242 more accurately.

The second image processor 314 is used to gradually adjust the initial pose (T1, R1) 317 to a navigation pose (Tnav, Rnav) 324 until a difference between the first image F1(x, y) 312 and a second image $F2_{Tnav, Rnav}$ (x, y) 322 falls in an allowable range. Herein the second image $F2_{Tnav, Rnav}$ (x, y) 322 is calculated based on the navigation pose (Tnav, Rnav) 324 and the predetermined model 318. In some embodiments, a second image $F2_{Tnav, Rnav}$ (x', y', z') in the special coordinate system (x', y', z') can be directly calculated based on the navigation pose (Tnav=[Tx', Ty', Tz'], Rnav=[Rx', Ry', Rz']) and the three dimension predetermined model 318. Then after a conversion from the special coordinate system (x', y', z') to the planar coordinate system (x, y), the second image $F2_{Tnav, Rnav}$ (x, y) 322 in the planar coordinate system (x,y) can be calculated.

In a more specific application, the second image processor 314 includes a second image calculator 308 and an image analysis unit 309. Referring to FIG. 4 and FIG. 5 together, the second image calculator 308 is used to calculate an initial second image $F2_{T1, R1}$ (x, y) 3221 based on the initial pose (T1, R1) 317 and the predetermined model 318. The second image calculator 308 is further used to calculate at least one adjusted second image 322 such as $F2_{T2, R2}$ (x, y) 3222, $F2_{T3, R3}$ (x, y) 3223 and $F2_{T4, R4}$ (x, y) 3224 based on a corresponding adjusted pose 323 (e.g., (T2, R2), (T3, R3) and (T4, R4)) calculated by the image analysis unit 309 and the predetermined model 318 if needed. As shown in FIG. 5, similar to the first image F1(x, y) 312, the second image 322 (e.g., the initial second image $F2_{T1, R1}$ (x, y) 3221) includes multiple corresponding feature points (e.g., 1', 2', 3',...n') of the internal element 501.

In some embodiments, the image analysis unit 309 is used to calculate an initial difference E(T1, R1) between the first image F1(x, y) 312 and the initial second image $F2_{T1, R1}$ (x, y) 3221. The image analysis unit 309 is further used to calculate an adjusted difference E(Tk+1, Rk+1) (k≥1) between the first image F1(x, y) 312 and the adjusted second image $F2_{Tk+1, Rk+1}$ (x, y) 322 such as $F2_{T2, R2}$ (x, y) 3222, $F2_{T3, R3}$ (x, y) 3223 and $F2_{T4, R4}$ (x, y) 3224. The image analysis unit 309 is further used to calculate a variation ΔEk between the initial difference E(T1, R1) and the adjusted difference E(Tk+1, Rk+1). The image analysis unit 309 is further used to determine whether the variation ΔEk falls in the allowable range, and gradually adjust the initial pose (T1, R1) 317 to the adjusted pose (Tk+1, Rk+1) 323 if the variation ΔEk falls out of the allowable range until the variation ΔEk falls in the allowable range. Once the variation ΔEk falls in the allowable range, the corresponding adjusted pose (Tk+1, Rk+1) 323 is outputted as the navigation pose (Tnav, Rnav) 324, such as (T4, R4) shown in FIG. 5 is the navigation pose 324 after the above calculation.

In other embodiments, the image analysis unit 309 is used to determine whether the difference E(T1, R1) between the first image 312 and the initial second image 3221 or the E(Tk+1, Rk+1) difference between the first image 312 and the adjusted second image 3222, 3223, 3224 falls in the allowable range, and gradually adjust the initial pose (T1, R1) 317 to the adjusted pose (Tk+1, Rk+1) 323 if the difference E(T1, R1), E(Tk+1, Rk+1) falls out of the allowable range until the difference E(T1, R1), E(Tk+1, Rk+1) falls in the allowable range. Once the difference E(T1, R1), E(Tk+1, Rk+1) falls in the allowable range, the initial pose (T1, R1) 317 or the corresponding adjusted pose (Tk+1, Rk+1) 323 is outputted as the navigation pose (Tnav, Rnav) 324, such as (T4, R4) shown in FIG. 5 is the navigation pose 324 after above calculation.

The navigation image calculator 310 is used to receive the navigation pose 324 and the predetermined model 318, and then calculate a navigation image 325 based on the navigation pose 324 and predetermined model 318. The illustrated navigation image 325 is a two-dimensional image, or a three-dimensional image. Then, the navigation image 325 is shown in the second part 226 of the display 220 to predict potential failure at the detected location and provide reference information to the operator for subsequent operation. In some embodiments, the navigation pose 324 is also shown in the display 220 to provide more reference information.

Referring to FIG. 5, a schematic view of an adjustment of an initial second image 3221 in accordance with one exemplary embodiment is shown. Combined with the block diagram of the borescope as shown in FIG. 4, the adjustment process will be described in detail as below.

In some embodiments, at least one adjustment process is implemented in the second image processor 314. The difference E(Tk, Rk) between the first image F1(x, y) 312 and the second image $F2_{Tk, Rk}$(x, y) 322 can be calculated according to the following equation:

$$\mathrm{E}(\mathrm{Tk}, \mathrm{Rk}) = \sum_{n=1}^{N} \left| F1(x. y) - F2_{\mathrm{Tk,Rk}}(x, y) \right|_n \ (k \geq 1) \tag{1},$$

Wherein the difference E(Tk, Rk) is calculated by an accumulation of the error between each point 1, 2, 3,...,n of F1(x, y) and the corresponding point 1', 2', 3',...n' of $F2_{Tk, Rk}$ (x, y). In other embodiments, E(Tk, Rk) is a function that can be used to describe the error between the first image F1(x, y) 312 and the second image $F2_{Tk, Rk}$ (x, y) 322.

As an example, the first image F1(x, y) and the initial second image $F2_{T1, R1}$ (x, y) 3221 are calculated as shown in the (a) part of FIG. 5. Then, an initial difference E(T1, R1) can be calculated according to the equation (1) when k=1.

The image analysis unit 309 is used to adjust the initial pose (T1, R1) 317 to an adjusted pose (T2, R2) 323.

The second image $F2_{T1, R1}$ (x, y) 3221 can be recalculated to $F2_{T2, R2}$ (x, y) 3222 based on the adjusted pose (T2, R2) 323 as shown in the (b) part of FIG. 5. Then, an E(T2, R2) can be calculated according to the equation (1) when k=2. A variation difference $\Delta E1 = E(T2, R2) - E(T1, T1)$ can be calculated. As an example, the $\Delta E1$ falls out of the predetermined allowable range (e.g., [-0.005, 0]), the adjusted pose (T2, R2) is determined not accurate still.

Then the image analysis unit 309 is used to adjust the initial pose (T1, R1) 317 to another adjusted pose (T3, R3) 323. The second image $F2_{T1, R1}$ (x, y) 3221 can be recalculated to $F2_{T3, R3}$ (x, y) 3223 as shown in the (c) part of FIG. 5. Then E(T3, R3) can be calculated based on the equation (1) when k=3. A variation difference $\Delta E2 = E(T3, R3) - E(T1, T1)$ can be calculated. As an example, the $\Delta E2$ falls out of the predetermined allowable range (e.g., [-0.005, 0]), the adjusted pose (T3, R3) 323 is determined not accurate still.

Then the image analysis unit 309 is used to adjust the initial pose (T1, R1) 317 to another adjusted pose (T4, R4) 323. The second image $F2_{T1, R1}$ (x, y) 3221 can be recalculated to $F2_{T4, R4}$ (x, y) 3224 as shown in the (d) part of FIG. 5. Then E(T4, R4) can be calculated based on the equation (1) when k=4. A variation difference $\Delta E3 = E(T4, R4) - E(T1, T1)$ can be calculated. As an example, the $\Delta E3$ falls in the predetermined allowable range (e.g., [-0.005, 0]), the adjusted pose (T4, R4) is determined accurate enough. Finally, the image analysis unit 309 outputs the adjusted pose (T4, R4) 323 as the navigation pose (Tnav, Rnav) 324.

In some embodiments, the adjusted pose (Tk+1, Rk+1) 323 is calculated by adding a compensation position $\Delta Tk$ and a compensation orientation $\Delta Rk$ to the initial position T1 and the initial orientation R1 respectively as the following equations.

$$Tk+1 = T1 + \Delta Tk \ (k \geq 1) \qquad (2),$$

$$Rk+1 = R1 + \Delta Rk \ (k \geq 1) \qquad (3).$$

In some embodiments, the step length of at least one of $\Delta Tk$ and $\Delta Rk$ are fixed and the direction of the at least one of $\Delta Tk$ and $\Delta Rk$ are variable. For example, $\Delta T1 = [0.005, -0.0005, 0.0005]$, $\Delta R1 = [0.5°, -0.5°, 0.5°]$ and $\Delta T2 = [0.005, -0.0005, -0.0005]$, $\Delta R2 = [0.5°, -0.5°, -0.5°,]$ . In some embodiments, $\Delta Tk$ and $\Delta Rk$ are variable. In some embodiments, $\Delta Tk$ and $\Delta Rk$ are calculated by a convergence algorithm such as the Levenberg-Marquard algorithm for accelerating a convergence speed of the difference E(Tk, Rk) to a value of zero.

In some embodiments, the adjusted difference E(Tk+1, Rk+1) is always expected to be less than the initial difference E(T1, R1). If E(Tk+1, Rk+1) is large than E(T1, R1), it means the adjustment is not in the right direction. In this case, the adjustment should change the direction, for example, $\Delta Tk = (0.0005, 0.0005, 0.0005)$ and $\Delta Tk+1 = (-00005, 0.0005, 0.0005)$. If E(Tk+1, Rk+1) is less than E(T1, R1) and $\Delta Ek$ remains out of the allowable range at the same time, it means the adjustment is in the right direction. The adjustment should continue or change the step length, for example, $\Delta Tk = (-0.0005, 0.0005, 0.0005)$ and $\Delta Tk+1 = (-0.0001, 0.0001, 0.0001)$.

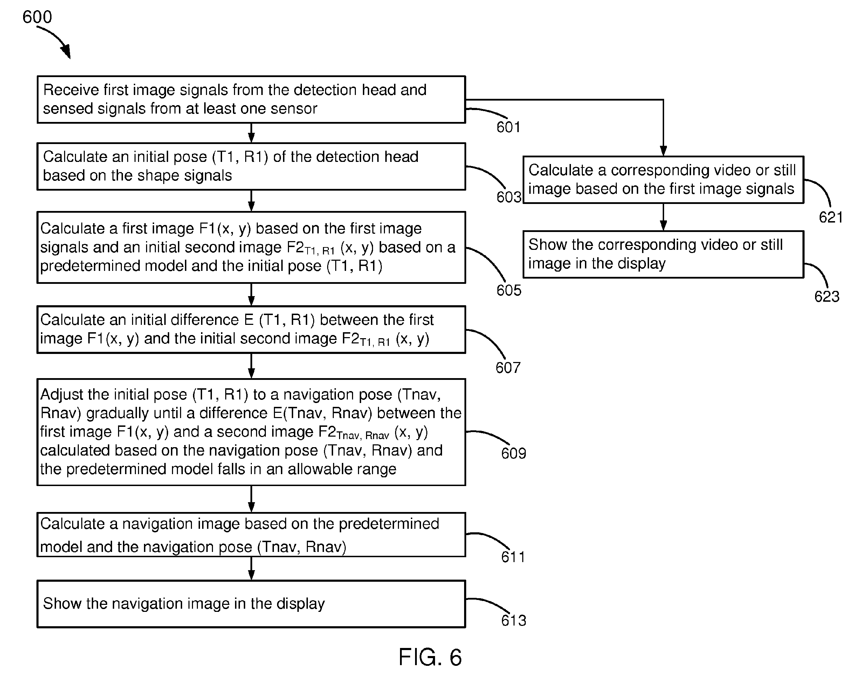

Referring to FIG. 6, a flowchart of a method for navigating a detection head 242 of the borescope 24 of FIG. 1 in accordance with one exemplary embodiment is shown. The method 600 is performed by the processor 300, and includes the following steps.

At block 601, during a detection operation, first image signals 311 are received from the detection head 242, and sensed signals 316 are received from the at least one sensor 2465.

For implementing the video or image viewing function, steps 621 and 623 are further included. At block 621, a corresponding video or still image 313 is calculated based on the first image signals 311. At block 623, the corresponding video or still image 313 is shown in the display 220.

For implementing the navigation function, steps 603 ~ 613 are included.

At block 603, an initial pose (T1, R1) 317 of the detection head 242 is calculated based on the sensed signals 316.

At block 605, a first image 312 is calculated based on the first image signals 311, and an initial second image 322 is calculated based on the initial pose (T1, R1) 317 and the predetermined model 318.

At block 607, an initial difference E (T1, R1) between the first image F1(x, y) 312 and the initial second image $F2_{T1, R1}$ (x, y) 3221 is calculated.

At block 609, the initial pose (T1, R1) 317 is gradually adjusted to a navigation pose (Tnav, Rnav) 324 until a corresponding difference E(Tnav, Rnav) between the first image F1(x, y) 312 and the second image $F2_{Tnav, Rnav}$ (x, y) 322 falls in an allowable range.

At block 611, a navigation image 325 is calculated based on the predetermined model 318 and the navigation pose (Tnav, Rnav) 324.

At block 613, the navigation image 325 is shown the display 220.

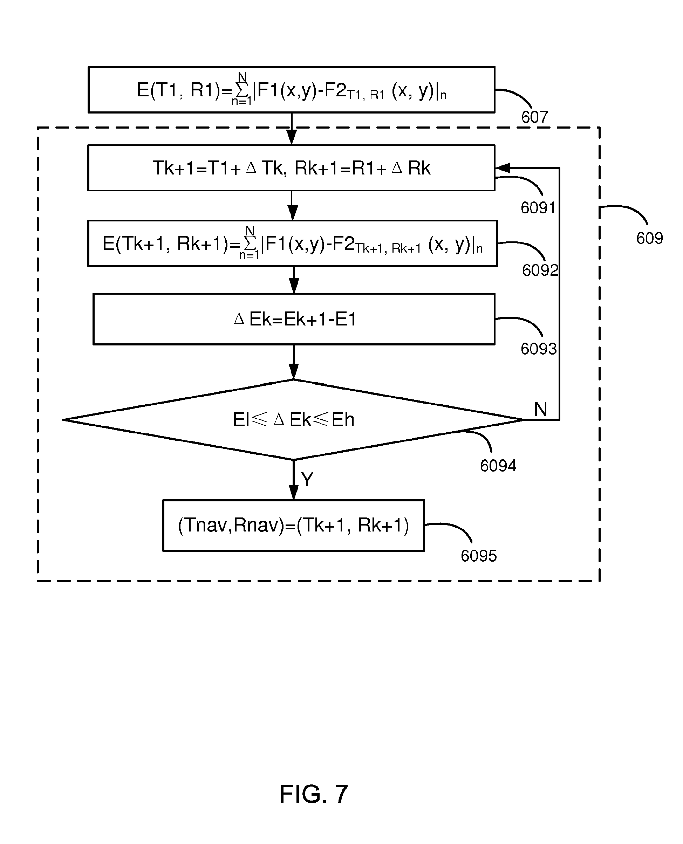

Referring to FIG. 7, a flowchart of a step for adjusting an initial pose of the method of FIG. 6 in accordance with one exemplary embodiment is shown. More specifically, the step 609 includes the following sub-steps.

At block 6091, the initial pose (T1, R1) 317 is adjusted to an adjusted pose (Tk+1, Rk+1)(k≥1)323.

At block 6092, the adjusted difference E(Tk+1, Rk+1) is calculated based on the first image F1(x, y) 312 and the adjusted second image $F2_{Tk+1, Rk+1}$ (x, y) 322. The adjusted second image $F2_{Tk+1, Rk+1}$(x, y) 322 is calculated based on the adjusted pose (Tk+1, Rk+1) 323.

At block 6093, a variation $\Delta Ek$ between the adjusted difference E(Tk+1, Rk+1) and the initial difference E(T1, R1) is calculated.

At block 6094, the variation $\Delta Ek$ is determined whether it falls in a predetermined range [El, Eh]. If not the process goes back to block 6091, if yes the process goes to block 6095.

At block 6095, the adjusted pose (Tk+1, Rk+1) 323 is outputted as the navigation pose (Tnav, Rnav) 324.

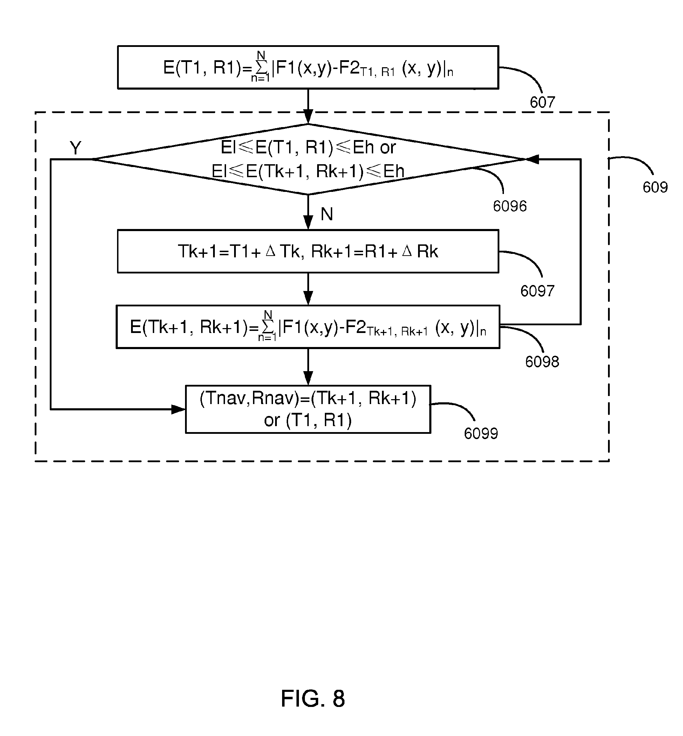

Referring to FIG. 8, a flowchart of a step for adjusting the initial pose of the method of FIG. 6 in accordance with another exemplary embodiment is shown. The step 609 includes the following sub-steps.

At block 6096, the initial difference E(T1, R1) or the adjusted difference E(Tk+1, Rk+1) (k≥1) is determined whether it falls in a predetermined range [El, Eh]. If not the process goes to block 6097, if yes the process goes to block 6099.

At block 6097, the initial pose (T1, R1) 317 is adjusted to an adjusted pose (Tk+1, Rk+1) 323.

At block 6098, the adjusted difference E(Tk+1, Rk+1) is calculated based on the first image F1(x, y) 312 and the adjusted second image $F2_{Tk+1, Rk+1}$ (x, y) 322. The adjusted second image $F2_{Tk+1, Rk+1}$(x, y) 322 is calculated based on the adjusted pose (Tk+1, Rk+1) 323. And the process goes back to block 6096.

At block 6099, the initial pose (T1, R1) 317 or the adjusted pose (Tk+1, Rk+1) 323 is outputted as the navigation pose (Tnav, Rnav) 324.

Since the initial pose (T1, R1) 317 is adjusted gradually, the corresponding second image 322 is adjusted accordingly. A difference between the first image 312 and the second image 322 is reduced gradually. Then an accurate navigation image 325 can be achieved through above method 600, the operator can accurately identify the interested detection channel/point through monitoring the navigation image 325 of the inserting tube 24 or the location of the detection head 242 in the detected mechanical device 70.

It is to be understood that a skilled artisan will recognize the interchangeability of various features from different embodiments and that the various features described, as well as other known equivalents for each feature, may be mixed and matched by one of ordinary skill in this art to construct additional systems and techniques in accordance with principles of this disclosure. It is, therefore, to be understood that the appended claims are intended to cover all such modifications and changes as fall within the true spirit of the invention.

Further, as will be understood by those familiar with the art, the present invention may be embodied in other specific forms without depending from the spirit or essential characteristics thereof. Accordingly, the disclosures and descriptions herein are intended to be illustrative, but not limiting, of the scope of the invention which is set forth in the following claims.

**Claims**

1. A borescope (20) comprising:

   an insertion tube (24) comprising a detection head (242) and at least one sensor (2465) for receiving signals in the insertion tube (24) and generating sensed signals (316);
   a first image processor (304) for calculating a first image (312) based on first image signals (311) captured by the detection head (242);
   a model store unit (306) for storing a predetermined model (318) of a mechanical device (70) to be detected;
   a pose calculator (307) for calculating an initial pose (317) of the detection head based (242) on the sensed signals (316);
   a second image processor (314) for adjusting the initial pose (317) to a navigation pose (324) until a difference between the first image (312) and a second image (322) calculated based on the navigation pose (324) and the predetermined model (318) falls in an allowable range;
   a navigation image calculator (310) for calculating a navigation image (325) based on the navigation pose (324) and the predetermined model (318); and
   a display (220) for showing the navigation image (325).

2. The borescope of claim 1, wherein the second image processor (314) comprises:

   a second image calculator (308) for:

calculating an initial second image (3221) based on the initial pose (317); and
calculating at least one adjusted second image (322) based on a corresponding adjusted pose (323) calculated by an image analysis unit (309) and the predetermined model (318); and

an image analysis unit (309) for:

calculating an initial difference between the first image (312) and the initial second image (3221);
calculating an adjusted difference between the first image (312) and the adjusted second image (3221);
calculating a variation ($\Delta EK$) between the initial difference and the adjusted difference;
determining whether the variation ($\Delta EK$) falls in the allowable range and gradually adjusting the initial pose (317) until the variation ($\Delta EK$) falls in the allowable range; and
outputting the corresponding adjusted pose (323) as the navigation pose (324).

3. The borescope of claim 1, wherein the second image processor (314) comprises:

a second image calculator (308) for:

calculating an initial second image (3221) based on the initial pose (317); and
calculating at least one adjusted second image (322) based on a corresponding adjusted pose (323) calculated by an image analysis unit (309) and the predetermined model (318); and

an image analysis unit (309) for:

determining whether the difference between the first image (312) and the initial second image (3221) or the difference between the first image (312) and the adjusted second image (322) falls in the allowable range, and gradually adjusting the initial pose (317) until the difference falls in the allowable range; and
outputting the corresponding adjusted pose (323) as the navigation pose (624).

4. The borescope of any of claims 1 to 3, wherein the initial pose (317) comprises an initial position (T1) and an initial orientation (R1) of the detection head (242).

5. The borescope of claim 4, wherein the adjusted pose (323) is obtained by adding a compensation position ($\Delta TK$) to the initial position (T1) or adding a compensation orientation ($\Delta RK$) to the initial orientation (R1).

6. The borescope of claim 5, wherein a step length of at least one of the compensation position ($\Delta TK$) and the compensation orientation ($\Delta RK$) are fixed.

7. The borescope of claim 5, wherein the compensation position ($\Delta TK$) and the compensation orientation ($\Delta RK$) are variable.

8. The borescope of claim 7, wherein the compensation position ($\Delta TK$) and the compensation orientation ($\Delta RK$) are calculated by a convergence algorithm for accelerating a convergence speed of the difference to a value of zero.

9. The borescope of claim 8, wherein the convergence algorithm comprises a Levenberg-Marquard algorithm.

10. The borescope of any preceding claim, wherein the sensed signals (316) comprise optical signals or stain change signals.

11. A method for navigating a detection head (242) of a borescope (20), the method comprising:

receiving first image signals (311) from the detection head (242) and sensed signals (316) from at least one sensor;
calculating an initial pose (317) of the detection head (242) based on the sensed signals (316);
calculating a first image (312) based on the first image signals (311) and an initial second image (3221) based on the initial pose (317) and a predetermined model (318);
calculating an initial difference between the first image (312) and the initial second image (3221);
adjusting the initial pose (317) to a navigation pose (324) gradually until a difference between the first image (312) and a second image (322) calculated based on the navigation pose (324) and the predetermined model falls (318) in an allowable range;
calculating a navigation image (325) based on the predetermined model (318) and the navigation pose (324); and
showing the navigation image (325).

**12.** The method of claim 11, further comprising:

calculating a corresponding video or still image (513) based on the first image signals (311); and
showing the corresponding video or still image. (513)

**13.** The method of claim 11 or 12, wherein the adjusting step comprises:

a) adjusting the initial pose (317) to an adjusted pose (323);
b) calculating the adjusted difference based on the first image (312) and a adjusted second image (323) based on the adjusted pose (323) and the predetermined model (318);
c) calculating a variation ($\Delta EK$) between the adjusted difference and the initial difference;
d) determining whether the variation ($\Delta EK$) falls in a predetermined range, if yes the process goes to step e), if not the process goes back to a); and
e) outputting the adjusted pose (323) as the navigation pose (324).

**14.** The method of claim 11 or 12, wherein the adjusting step comprises:

a) determining whether an initial difference or an adjusted difference between the first image (312) and the second image (322) falls in a predetermined range, if not the process goes to step b), if yes the process goes to step d);
b) adjusting the initial pose (317) to an adjusted pose (323);
c) calculating the adjusted difference based on the first image (312) and the adjusted second image (3221) calculated based on the adjusted pose (323) and the predetermined model (318), and then the process goes back to step a); and
d) outputting the initial pose (317) or the adjusted pose (323) as the navigation pose (324).

**15.** The method of any of claims 11 to 14, wherein the initial pose (317) comprises an initial position (T1) and an initial orientation (R1) of the detection head (242) and wherein the adjusted pose (323) is obtained by adding a compensation position ($\Delta TK$) to the initial position (T1) and adding a compensation orientation ($\Delta RK$) to the initial orientation (R1).

FIG. 1

FIG. 2

FIG. 3

FIG. 4

312

F1(x, y)

F2 T1, R1 (x,y)

3221

(a)

312

F1(x, y)

F2 T2, R2 (x,y)

3222

(b)

312

F1(x, y)

F2 T3, R3 (x,y)
3223

(c)

312

F1(x, y)

F2 T4, R4 (x,y)

3224

(d)

FIG. 5

```
┌─────────────────────────────────────────────────────┐
│ Receive first image signals from the detection head  │
│ and sensed signals from at least one sensor          │──── 601
└─────────────────────────────────────────────────────┘
                        │
                        ▼
┌─────────────────────────────────────────────────────┐
│ Calculate an initial pose (T1, R1) of the detection  │
│ head based on the shape signals                      │──── 603
└─────────────────────────────────────────────────────┘
                        │
                        ▼
┌─────────────────────────────────────────────────────┐
│ Calculate a first image F1(x, y) based on the first  │
│ image signals and an initial second image            │
│ F2_{T1, R1} (x, y) based on a predetermined model    │──── 605
│ and the initial pose (T1, R1)                        │
└─────────────────────────────────────────────────────┘
                        │
                        ▼
┌─────────────────────────────────────────────────────┐
│ Calculate an initial difference E (T1, R1) between   │
│ the first image F1(x, y) and the initial second      │──── 607
│ image F2_{T1, R1} (x, y)                             │
└─────────────────────────────────────────────────────┘
                        │
                        ▼
┌─────────────────────────────────────────────────────┐
│ Adjust the initial pose (T1, R1) to a navigation     │
│ pose (Tnav, Rnav) gradually until a difference       │
│ E(Tnav, Rnav) between the first image F1(x, y) and a │
│ second image F2_{Tnav, Rnav} (x, y) calculated       │──── 609
│ based on the navigation pose (Tnav, Rnav) and the    │
│ predetermined model falls in an allowable range      │
└─────────────────────────────────────────────────────┘
                        │
                        ▼
┌─────────────────────────────────────────────────────┐
│ Calculate a navigation image based on the            │
│ predetermined model and the navigation pose          │──── 611
│ (Tnav, Rnav)                                         │
└─────────────────────────────────────────────────────┘
                        │
                        ▼
┌─────────────────────────────────────────────────────┐
│ Show the navigation image in the display             │──── 613
└─────────────────────────────────────────────────────┘
```

From 601:
```
┌─────────────────────────────────────────────────────┐
│ Calculate a corresponding video or still image based │
│ on the first image signals                           │──── 621
└─────────────────────────────────────────────────────┘
                        │
                        ▼
┌─────────────────────────────────────────────────────┐
│ Show the corresponding video or still image in the   │
│ display                                              │──── 623
└─────────────────────────────────────────────────────┘
```

FIG. 6

$$E(T1, R1) = \sum_{n=1}^{N} |F1(x,y) - F2_{T1, R1}(x, y)|_n$$

607

$$Tk+1 = T1 + \Delta Tk, \quad Rk+1 = R1 + \Delta Rk$$

6091

$$E(Tk+1, Rk+1) = \sum_{n=1}^{N} |F1(x,y) - F2_{Tk+1, Rk+1}(x, y)|_n$$

6092

$$\Delta Ek = Ek+1 - E1$$

6093

609

$$El \leqslant \Delta Ek \leqslant Eh$$

N

6094

Y

$$(Tnav, Rnav) = (Tk+1, Rk+1)$$

6095

FIG. 7

$$E(T1, R1) = \sum_{n=1}^{N} |F1(x,y) - F2_{T1, R1}(x, y)|_n$$

607

$$El \leq E(T1, R1) \leq Eh \text{ or}$$
$$El \leq E(Tk+1, Rk+1) \leq Eh$$

Y

N

6096

609

$$Tk+1 = T1 + \triangle Tk, \quad Rk+1 = R1 + \triangle Rk$$

6097

$$E(Tk+1, Rk+1) = \sum_{n=1}^{N} |F1(x,y) - F2_{Tk+1, Rk+1}(x, y)|_n$$

6098

$$(Tnav, Rnav) = (Tk+1, Rk+1)$$
$$\text{or } (T1, R1)$$

6099

FIG. 8

Europäisches
Patentamt

European
Patent Office

Office européen
des brevets

## EUROPEAN SEARCH REPORT

Application Number

EP 15 16 5769

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/292166 A1 (ITO SEIICHI [JP] ET AL) 26 November 2009 (2009-11-26) <br> * figures 1-4,8-11,15,19-24 * <br> * paragraph [0037] - paragraph [0083] * <br> ----- | 1-15 | INV. <br> G02B23/24 <br> G01N21/954 <br> A61B1/00 |
| X | US 2012/289843 A1 (CHOPRA PRASHANT [US] ET AL) 15 November 2012 (2012-11-15) <br> * figures 1,2,4,5,8-12,16-22 * <br> * paragraph [0034] - paragraph [0037] * <br> * paragraph [0048] - paragraph [0062] * <br> * paragraph [0066] - paragraph [0100] * <br> ----- | 1-15 | |
| A | DE 10 2011 122759 A1 (ROLLS ROYCE DEUTSCHLAND [DE]) 2 May 2013 (2013-05-02) <br> * the whole document * <br> ----- | 1,11 | |
| A | US 2013/303891 A1 (CHOPRA PRASHANT [US]) 14 November 2013 (2013-11-14) <br> * figures 2,4-6 * <br> ----- | 1,11 | |
| A | US 2009/149703 A1 (TANAKA HIDEKI [JP]) 11 June 2009 (2009-06-11) <br> * figures 1-33 * <br> ----- | 1,11 | TECHNICAL FIELDS SEARCHED (IPC) <br><br> G02B <br> G01N <br> A61B |

The present search report has been drawn up for all claims

| Place of search | Date of completion of the search | Examiner |
|---|---|---|
| Munich | 18 September 2015 | Beutter, Matthias |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.................................................................

& : member of the same patent family, corresponding
document

EPO FORM 1503 03.82 (P04C01)

**ANNEX TO THE EUROPEAN SEARCH REPORT
ON EUROPEAN PATENT APPLICATION NO.**                    EP 15 16 5769

This annex lists the patent family members relating to the patent documents cited in the above-mentioned European search report.
The members are as contained in the European Patent Office EDP file on
The European Patent Office is in no way liable for these particulars which are merely given for the purpose of information.

18-09-2015

| Patent document cited in search report | | Publication date | Patent family member(s) | | Publication date |
|---|---|---|---|---|---|
| US 2009292166 | A1 | 26-11-2009 | EP | 2123216 A1 | 25-11-2009 |
| | | | JP | 5372407 B2 | 18-12-2013 |
| | | | JP | 2009279250 A | 03-12-2009 |
| | | | US | 2009292166 A1 | 26-11-2009 |
| US 2012289843 | A1 | 15-11-2012 | US | 2012289843 A1 | 15-11-2012 |
| | | | WO | 2013074726 A1 | 23-05-2013 |
| DE 102011122759 | A1 | 02-05-2013 | DE | 102011122759 A1 | 02-05-2013 |
| | | | WO | 2013064242 A2 | 10-05-2013 |
| US 2013303891 | A1 | 14-11-2013 | EP | 2849668 A1 | 25-03-2015 |
| | | | US | 2013303891 A1 | 14-11-2013 |
| | | | WO | 2013173234 A1 | 21-11-2013 |
| US 2009149703 | A1 | 11-06-2009 | EP | 1917901 A1 | 07-05-2008 |
| | | | US | 2009149703 A1 | 11-06-2009 |
| | | | WO | 2007023631 A1 | 01-03-2007 |

EPO FORM P0459

For more details about this annex : see Official Journal of the European Patent Office, No. 12/82